(19)

Europäisches Patentamt

European Patent Office

Office européen des brevets

(11) **EP 2 537 035 B1**

(12) **EUROPEAN PATENT SPECIFICATION**

(45) Date of publication and mention
of the grant of the patent:
**16.11.2016 Bulletin 2016/46**

(21) Application number: **11704217.6**

(22) Date of filing: **16.02.2011**

(51) Int Cl.:
***G01N 33/68*** (2006.01)

(86) International application number:
**PCT/EP2011/052282**

(87) International publication number:
**WO 2011/101370 (25.08.2011 Gazette 2011/34)**

(54) **Method for the determination of sequence variants of polypeptides**

Verfahren zur Bestimmung von Sequenzvarianten von Polypeptiden

Procédé pour déterminer les variantes de séquences de polypeptides

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.02.2010 EP 10001645**

(43) Date of publication of application:
**26.12.2012 Bulletin 2012/52**

(73) Proprietor: **F.Hoffmann-La Roche AG**
**4070 Basel (CH)**

(72) Inventors:
• **KOLL, Hans**
**85247 Schwabhausen (DE)**
• **REGULA, Joerg Thomas**
**81377 Muenchen (DE)**
• **WIEGESHOFF, Frank**
**67063 Ludwigshafen (DE)**
• **ZECK, Anne**
**72768 Reutlingen (DE)**

(74) Representative: **Burger, Alexander**
**Roche Diagnostics GmbH**
**Patent Department (LPP.....6164)**
**P.O.Box 11 52**
**82372 Penzberg (DE)**

(56) References cited:
• **SREBALUS BARNES C A ET AL: "Applications of mass spectrometry for the structural characterization of recombinant protein pharmaceuticals", MASS SPECTROM REV, vol. 26, no. 3, May 2007 (2007-05), pages 370-388, XP002586634, cited in the application**
• **ZHANG Z ET AL: "Mass spectrometry for structural characterization of therapeutic antibodies.", MASS SPECTROM REV, vol. 28, no. 1, January 2009 (2009-01), pages 147-176, XP002586635, cited in the application**
• **YU X C ET AL: "Identification of codon-specific serine to asparagine mistranslation in recombinant monoclonal antibodies by high-resolution mass spectrometry.", ANAL CHEM, vol. 81, no. 22, 15 November 2009 (2009-11-15), pages 9282-9290, XP002586636, cited in the application**
• **HOUDE D ET AL: "Determination of protein oxidation by mass spectrometry and method transfer to quality control", J CHROMATOGR, vol. 1123, no. 2, 11 August 2006 (2006-08-11), pages 189-198, XP024967762, cited in the application**
• **SWARTZ M ET AL: "Validation and Peptide Mapping", Chromatography Online, May 2007 (2007-05), pages 1-5, XP002586637, Retrieved from the Internet: URL:http://chromatographyonline.findanalyt ichem.com/lcgc/LC%2FHPLC/Validation-and-Pe ptide-Mapping/ArticleStandard/Article/deta il/429508 [retrieved on 2010-06-10]**

Remarks:
The file contains technical information submitted after the application was filed and not included in this specification

EP 2 537 035 B1

## Description

Herein is reported a method for the determination of sequence variants of a polypeptide comprising a tryptic digestion of the polypeptide, a chromatographic separation of the fragments, high resolution mass spectrometry of the separated fragments and determination of sequence variants.

## Background of the Invention

Proteins play an important role in today's medical portfolio. For human application every therapeutic protein has to meet distinct criteria. To ensure the safety of biopharmaceutical agents to humans the characteristics of the therapeutic protein have to be within certain limits and by-products accumulating during the production process have to be removed especially.

Any change in or modification of the amino acid composition and sequence, respectively, of a polypeptide, which differs from the desired form is defined as a sequence variant. This can be a nucleic acid mutation resulting in a change of the amino acid sequence, undesired post-translational modifications (PTMs) or aberrant polypeptide variants (shortened or elongated forms).

To guarantee polypeptide consistency and to avoid errors, the influence of biological mechanisms (e.g. fidelity of replication, accuracy of transcription and translation) and technical processes (transfection and amplification in cell line development or fermentative conditions) on the presence of sequence variants have to be assessed. Also a method providing high sensitivity to find and identify potential sequence variants has to be provided.

For example, cell line development processes can result in sequence variants which may become critical for cell line switches and clone changes, respectively. Fermentation under unexpected shortage of certain media components in the feeding media and down stream processing (DSP) may cause sequence variants or post translational modification of amino acids, respectively. Therefore, the presence of sequence variants has to be checked to confirm product homogeneity and consistency.

Barnes, C.A.S., et al., Mass. Spectrom. Rev. 26 (2007) 370-388 report applications of mass spectrometry for the structural characterization of recombinant protein pharmaceuticals. Mass spectrometry for structural characterization of therapeutic antibodies is reported by Zhang, Z., et al., in Mass. Spectrom. Rev. 28 (2009) 147-176. Yu, X.C., et al. (Anal. Chem. 81 (2009) 9282-9290) report the identification of codon-specific serine to asparagine mistranslations in recombinant monoclonal antibodies by high-resolution mass spectrometry. The determination of protein oxidation by mass spectrometry and method transfer to quality control is reported by Houde, D., et al., J. Chromatogr. 1123 (2006) 189-198.

## Summary of the Invention

Herein is provided a method for the determination of amino acid sequence variants, i.e. mutations in the amino acid sequence, of a polypeptide. Indirectly therewith also nucleic acid sequence variants can be determined.

In more detail herein is reported a method for determining amino acid sequence mutations in a (produced) polypeptide, comprising the following steps:

a) providing a sample of a (produced) polypeptide, b) incubating the polypeptide in the sample with a protease, c) performing a two dimensional analysis using reversed phase chromatography coupled with high resolution mass spectroscopy (FT-ICR / FT-orbitrap) and MS/MS analysis of the amino acid sequence fragments of the peptides, d) data evaluation by comparing the LC-MS data sets obtained for the samples side by side with the data set of a reference sample, by searching for differences in the signal intensities at given retention times and by evaluation of differential signals with respect to amino acid sequence mutations. The reference sample for data evaluation (step d) can be either a well characterized standard or one of the samples to be analyzed.

A first aspect as reported herein is a method for determining an amino acid sequence variant, especially an amino acid mutation in the amino acid sequence, of a polypeptide, characterized in comprising the following steps:

a) providing at least two samples of the polypeptide,
b) incubating the samples each with a protease,
c) analyzing the incubated samples by a two dimensional data analysis comprising the combination of reversed phase chromatography separation with mass spectrometry analysis and/or MS/MS analysis,
d) defining the data-set obtained with one sample in step c) as reference sample and comparing the data-sets obtained with the other samples in step c) with the data-set of the reference sample, whereby every difference determined is an amino acid sequence variation (mutation) of the polypeptide and thereby determining an amino acid sequence variant of the polypeptide.

Another aspect as reported herein is a method for determining an amino acid sequence mutation in the amino acid sequence of a polypeptide, characterized in comprising the following steps:

a) providing at least two samples of the polypeptide,
b) incubating the samples each with a protease,
c) analyzing the incubated samples by a two dimensional data analysis comprising the combination of reversed phase chromatography separation with mass spectrometry analysis and/or MS/MS analysis,
d) defining the data-set obtained with one sample in step c) as reference sample and comparing the data-sets obtained with the other samples in step c) with the data-set of the reference sample, whereby every difference determined is an amino acid sequence mutation of the polypeptide and thereby determining an amino acid sequence mutation of the polypeptide,

or the following steps:

a) providing at least one sample of the polypeptide,
b) incubating the sample with a protease,
c) analyzing the incubated sample by a two dimensional data analysis comprising the combination of reversed phase chromatography separation with mass spectrometry analysis and/or MS/MS analysis,
d) comparing the data-set obtained with the sample in step c) with the data-set of a reference sample determined with a method as reported in steps b) and c), whereby every difference determined is an amino acid sequence mutation of the polypeptide and thereby determining an amino acid sequence mutation of the polypeptide.

The provided samples may originate from different clones obtained by transient transfection of a nucleic acid encoding the polypeptide, or from stable transfected cell lines, or from cell lines of different age, or different fermentation scales, or fermentation runs under different conditions. In one embodiment of from 2 to 10,000 samples are provided, in another embodiment of from 2 to 1,000 samples are provided, and in a further embodiment of from 2 to 348 samples are provided.

In one embodiment the method further comprises a step

e) determining the identity and position of the amino acid sequence variation (mutation) by MS/MS fragmentation and data analysis.

In another embodiment an additional sample is provided which comprises the polypeptide spiked with the polypeptide with a known amino acid sequence variation (mutation). The additional sample is incubated, analyzed and compared in addition to the provided samples. In one embodiment the analyzing is performed at a pH value of less than 8.0 and at a temperature of less than 40 °C. These conditions reduce method induced amino acid sequence changes. In a further embodiment the pH value is of from pH 6.5 to less than pH 8.0. In another embodiment the temperature is of from 20 °C to 40 °C. In another embodiment the sample is provided in a tris (hydroxymethyl) aminomethane buffer for enzymatic digestion. In still a further embodiment the incubating of the samples with a protease is a cleavage of the polypeptide by the protease in fragments of from 3 to 60 amino acid residues in length.

In one embodiment the comparing in step d) is performed with the data of one, or some, or all MS-charge states. In a further embodiment the comparing comprises the overlaying of the mass spectrometry total ion chromatograms (MS-TIC) of the reference sample and each of the other samples, whereby the intensity ratio of all overlapped and aligned masses is calculated and peaks with a ratio of more than 10 have to be considered as amino acid sequence variation (amino acid sequence mutation). In still a further embodiment the comparing comprises in addition the comparing of the DNA translated proteolytic fragment peptide pattern to the mass spectrometry total ion chromatogram (MS-TIC) of the sample. For amino acid sequence variant (amino acid sequence mutation) search single base substitutions, deletions and insertions in the encoding nucleic acid sequence of the polypeptide investigated are allowed, obtained sequences are in silico translated and in silico digested with the same enzyme as used in the method. Subsequently, amino acid sequence variants (amino acid sequence mutations) are identified by matching the experimentally determined MS/MS fragment spectrum of a peptide with the theoretical MS/MS spectra of the peptide derived from the in silico process described before.

In a further embodiment the polypeptide is a complete immunoglobulin, an immunoglobulin fragment, or an immunoglobulin conjugate. In another embodiment the polypeptide in the samples is reduced and the free sulfhydryl residues are carboxymethylated prior to the incubation with the protease.

The samples are all treated separately, i.e. individually and not as mixture, in the steps according to the methods as reported herein. In one embodiment the samples are treated individually in the reducing step, the incubating step, and the analyzing step. In one embodiment the method is a method for high throughput determination.

In one embodiment the samples are incubated for 16 hours to 18 hours with the protease and immediately thereafter formic acid or trifluoro acetic acid are added. In a further embodiment the samples are incubated for 4 hours with the protease and immediately thereafter formic acid or trifluoro acetic acid are added.

Another aspect as reported herein is a method for producing a polypeptide comprising the step of selecting a cell producing a polypeptide, whereby the polypeptide comprises the smallest number and the smallest ratio, respectively, of amino acid sequence variations, i.e. mutations in the amino acid sequence, of all processed samples. Another aspect as reported herein is a method for producing a polypeptide comprising the step of selecting a cell producing a polypeptide, whereby the polypeptide comprises no detectable amino acid sequence mutation (variations). In one embodiment the smallest number and the smallest ratio, respectively, of amino acid sequence variations (mutations in the amino acid sequence) is determined with respect to a reference sample or a predetermined amino acid sequence.

In one example the method comprises the steps of:

a) providing at least two cells comprising a nucleic acid encoding the polypeptide,
b) single depositing and cultivating the cells,
c) performing with the single deposited cells a method as reported herein,
d) selecting a cell producing a polypeptide, whereby the polypeptide comprises the smallest number, and/or smallest ratio of amino acid sequence variations (amino acid sequence mutations),
e) cultivating the selected cell,
f) producing a polypeptide by recovering the polypeptide from the cell or the cultivation medium.

In one embodiment the provided cell is selected from a transiently transfected cell, or a stable transfected cell, or an immune cell obtained from an animal after immunization.

## Detailed Description of the Invention

Therapeutic proteins, e.g. produced by recombinant methods, may be a mixture of molecules with slightly different amino acid sequences, whereby the difference is not only at the C- or N-terminus of the amino acid sequence but also within the amino acid sequence (e.g. amino acid mutations, exchanges, oxidation or thioether formation). Amino acid sequence mutations (variations) can be detected with a method as reported herein by peptide mapping methodology. These amino acid sequence mutations (variations) may emerge, for example, i) during nucleic acid replication by errors of the nucleic acid polymerase, or ii) during nucleic acid transcription by errors of the RNA polymerase or the splicing apparatus, or iii) during protein translation by acquisition of the wrong t-RNA or by t-RNAs loaded with the wrong amino acid, or iv) as unintended post-translational modification or incomplete removal of the signal peptide. Therefore, an amino acid sequence mutation (variation) can result from the integration/amplification process of the DNA encoding the respective polypeptide, or from the replication of the coding nucleic acid, or the transcription including post transcriptional modification (e.g. during mRNA splicing or RNA editing) or during translation. Amino acid variations resulting from a modification of individual amino acid residues after release of the molecule from the ribosome are defined as not necessarily unintended post translational modification and are not a mutation (variant) as termed herein.

From literature it is known that eukaryotic (as well as prokaryotic) enzymes have an average error rate of:

replication:  -1 per $10^8$ to $10^{12}$ replicated nucleic acid bases by normal polymerases,
-1 per $10^1$ to $10^3$ replicated nucleic acid bases by error-prone polymerases;

transcription:  1 per $10^4$ to $10^5$ transcribed nucleic acid base pairs;
.translation:  1 per $10^3$ to $10^4$ incorporated amino acid residues.

This shows that on the one hand the error rates during the replication step by the induction of error-prone polymerases and on the other hand during translation have the most influence especially in combination with the "stress" exerted on the cells, e.g. during the selection process after transfection or during the growth in the presence of a selection agent. Additionally mutations during translation are statistical events whereas mutations during replication would result in single events with stable location/locus. That is, errors during replication would result in position specific and reproducible amino acid sequence mutations (differences), whereas transcriptional and translational errors can result in non-position specific amino acid mutations (variations) and a statistical distribution of these changes over the entire molecule. Finally, also the transfection process including integration into the genome for obtaining the recombinant cell can results in up to 1 % changed DNA (see e.g. Lebkowski, J. S., et al., Mol. Cell Biol. 4 (1984) 1951-1960). This would result in a position specific mutation (variation) prior to replication with a frequency of up to 1:100.

Herein is reported a sample preparation, data collection and evaluation method using two dimensional data analysis comprising a LC-MS/MS based peptide mapping with highest possible sequence coverage in order to prove the correct composition and amino acid sequence of a polypeptide and the relative quantification of potentially mutant amino acid sequences (variants) vs. non-mutant amino acid sequences (non-variants). It has to be pointed out that the method as reported herein uses fragmented proteins instead of intact or complete proteins for the analysis. This increases the sensitivity of the method to the detection of very low levels of mutations. This also avoids the interference and the need of the distinguishing and the resolving of isobaric masses for possible mutations.

For the determination of an amino acid sequence mutation (variation) two different starting positions are possible:

Case A: If already a characterized sample is available this can be chosen as reference sample and the sample to be determined can be compared therewith: the known peptide pattern and peak assignment is used as reference and every detected difference in the sample can be an amino acid sequence mutation (variation). Such a reference material may be a material from a well characterized cell line, e.g. from a defined fermentation process.

Case B: If no already characterized sample is available then

a) for peak assignment a complete characterization of one sample can be performed and Case A can be applied/followed (Case B-1); or
b) one sample is arbitrarily chosen as reference sample (Case B-2) and the remaining samples are compared therewith; this includes the determination of differences, i.e. mutations, in both, the reference sample as well as the non-reference samples.

The reference sample may be from a different cell line, or cell line generation, or cultivation scale, or obtained under changed/different cultivation conditions (such as e.g. media composition).

In one embodiment if a large number of samples have to be analyzed any sample can be chosen as reference sample and all other samples can be grouped according to the determined amino acid sequence mutations (variation). Afterwards a detailed characterization is performed in each group.

In one embodiment of all aspects as reported herein the polypeptide is a complete immunoglobulin, or an immunoglobulin fragment, or an immunoglobulin conjugate.

**General Methodology:**

A sample containing the polypeptide to be analyzed is digested with a protease, e.g. trypsin, resulting in characteristic amino acid sequence fragments (peptides). In one embodiment the size of the amino acid sequence fragments is starting of from 3 or 4 amino acid residues and up to 60 amino acid residues in length. In the second step a chromatographic separation of the amino acid sequence fragments (reversed phase high performance liquid chromatography, RP-HPLC) coupled with high resolution mass spectrometry (MS) using Fourier transform ion cyclotron (FT-ICR / FT-orbitrap) technology and mass spectrometry (MS/MS) analysis of the amino acid sequence fragments obtained in the mass spectrometer by collisionally induced dissociation (CID) is performed. This is a two dimensional data analysis. Due to the two dimensions of the analysis, i.e. time versus mass, an improved resolution can be obtained. The two dimensional analysis also allows that a low resolution of the liquid chromatography separation can be accepted prior to the MS analysis as overlapping peaks can be resolved during the MS analysis.

The obtained HPLC-MS data sets are thereafter compared, i.e. a comparison of amino acid sequence fragment elution patterns and mass to charge (m/z) patterns of the samples and identification of mutations, i.e. differences, is performed by analysis of the MS/MS fragment spectra. If a reference sample based analysis is performed, amino acid fragment identification, peak assignment and/or determination of sequence coverage have to be performed in addition to the detection of amino acid sequence fragments containing amino acid sequence mutations (variations) and their relative quantification. In one embodiment the polypeptide is either an immunoglobulin or a non-immunoglobulin polypeptide.

With the method as reported herein the composition of a sample and the fraction of molecules with amino acid sequence mutations (variations) therein can be determined. Also a qualitative determination of the consistency of a sample can be performed by matching the experimentally determined mass spectrometry fragmentation data to theoretical amino acid sequence fragment masses obtained by in silico proteolytic digestion of the polypeptide and its variants and subsequent simulation of collisionally induced fragmentation. Finally a sequence with proposed mutations (variations) and mutation (variant) positions can be obtained and confirmed by manual data evaluation. Once an amino acid sequence mutation (variation) has been identified it can be quantified relative to the unmodified amino acid sequence by using the data of one, some, or all MS-charge states.

It has been found that by employing spiking experiments the method as reported herein has an overall limit of detection of at least 0.1-1.0 % of mutant amino acid sequence (variants). It has further been found that it is on one hand advantageous to avoid basic pH values and high temperatures during the sample preparation in order to avoid deamidation, i.e. method induced modifications. Therefore in one embodiment of the method as reported herein the method is performed in the neutral and weak basic pH range, i.e. at a pH vale below pH 8.0 and above pH 6.5, and at moderate temperatures, i.e. at temperatures below 40 °C. Further has been found that as in one embodiment by using a tris (hydroxymethyl) aminomethane buffer instead of a commonly used ammonia-bicarbonate buffer for mass analysis less method dependent artifacts are obtained.

The current method uses LC-MS data sets for the determination and allows for the detection and identification of one or more amino acid mutations (variations) in a tryptic digest of a sample polypeptide. For the identification MS/MS data can be used and the "error-tolerant-search" method identifies amino acid sequence mutations (variations) based on a single nucleic acid exchange, insertion or deletion. Analogously it is also possible to identify fragments and some other known modifications.

This comparative approach can dramatically reduce the required time for the analysis as only the differences have to be specifically analyzed. Also, if selected, the reference sample has to be analyzed only once. If many mutations (differences) are present in the samples these can be grouped by modifications and representative samples of each group can be analyzed.

**Detailed Method:**

In the following specific embodiment of all methods as reported herein are described.

The samples can be reduced by the addition of dithiotreitol (DDT). Also free sulfhydryl residues can be carboxymethylated by iodoacetic acid. Afterwards the buffer of the sample can be exchanged and adjusted for the enzymatic digestion. The sample can be enzymatically digested overnight (16 to 18 hours) and the digestion can be stopped by the addition of trifluoro acetic acid. The digested sample can be subjected to RP-HPLC separation and high resolution mass spectrometric analysis of the separated amino acid sequence fragments (using e.g. FT-ICR/FT-orbitrap mass spectrometer).

The term "data set" as used herein denotes the mass to charge ratios obtained time-resolved during the chromatographic elution in a mass spectrometer by ionizing the digested and time resolved amino acid sequence fragments contained in a sample in order to generate charged molecules or charged fragments. The data set comprises at least the mass spectrometric total ion chromatogram (MS-TIC) and the tandem mass spectrometric data (MS/MS-data) obtained by collisionally induced dissociation of the parent molecules.

**Case A:**

Samples to be assessed are analyzed together with an available reference sample. Each sample and the reference sample should be determined at least twice. As positive control a sample which is similar to the reference sample, i.e contains one or two amino acid sequence mutations (variations), is spiked to the reference sample and used as artificial mutant amino acid sequence (variant). The artificial mutant amino acid sequence (variant) is spiked to the reference sample, in one embodiment in 0.5 % (w/w), for confirmation of the overall sensitivity of the method. For setting up the parameters of the analytical method the results obtained with the reference sample, i.e. the recombinantly produced polypeptide without a mutation (modification) or with known mutation (modification) pattern, and with the reference sample spiked with the mutant amino acid sequence with the artificial amino acid sequence mutation (variant) can be compared. With these parameters the following analysis can be carried out.

For the determination the total ion chromatogram (TIC) of the reference sample and a sample to be analyzed can be overlaid and the signal intensities of corresponding mass signals at a given retention time (i.e. retention time and mass related) can be compared. Thus, in one embodiment the analyzing and/or the determining is a two dimensional analyses using a separation according to chromatographic retention time and mass (m/z value). The intensity ratios, i.e. the ratios of the signal intensities of the sample to the signal intensities of the reference sample, of all overlaid/aligned masses can be calculated. This ratio can be plotted against the retention time, whereby:

a) peaks of the same mass and with the same intensity in reference sample and sample are denoted with values of about 1, whereby masses present more frequently in the sample to be analyzed are denoted with values larger than 1,

b) all masses with a value of more than 1, in one embodiment with a value of more than 3, or more than 10, or more than 100, in one embodiment with a value of from 10 to $5*10^9$, in another embodiment with a value of from 100 to $5*10^9$, are identified, whereby for the identification all MS/MS spectra corresponding to the parent mass are used and the different charge states are also taken into account,

c) quantification of a mutant amino acid sequence (variation) can be carried out by using one, or some, or all charge states in the mass spectrum relative to each other, whereby for the natural amino acid sequence and the mutant amino acid sequence (variant) the same charge state is chosen, the extracted ion chromatograms (EICs) are generated and the area under the curve of the peaks corresponding to each other in the EICs are quantified relative to each other according to the following formula:

$$\frac{100}{\text{natural amino acid sequence} + \text{mutant amino acid sequence}} * \text{mutant amino acid sequence}$$

In all embodiments the m/z-window used for the analyzing is the respective mass plus/minus 1.6 amu m/z. In order to ensure that the data can be processed in a timely manner in all embodiments a ratio of 3 is set as threshold, i.e. all ratios below that limit are not analyzed.

**Case B:**

In this case no already characterized sample is available. For one of the provided samples to be analyzed (in this case more than one sample to be analyzed has to be available) an alignment of all determined masses in the TIC to the theoretically determined peptide pattern can be performed, i.e. a peak assignment can be performed (with predetermined protease for the digest). Important for the correct alignment can be on the one hand the exact mass and on the other hand the MS/MS fragment coverage for sequence confirmation of the peptide suggestions. In one specific embodiment amino acid sequence mutations (variations) can be identified by an error-tolerant search by adding to or subtracting from, respectively, each calculated theoretical mass of the natural amino acid sequence the mass differences resulting from a nucleic acid mutation, i.e. a single nucleotide substitution, deletion, or insertion in a base triplet (codon) resulting in a difference in the amino acid sequence. Subsequently, a suggested amino acid sequence mutation (variation) has to be confirmed by the MS/MS-fragmentation pattern. Therewith not only the identity of the amino acid sequence mutation (difference) but also the position of the amino acid mutation (change) has to be covered by the masses of the fragments in the MS/MS-analysis.

However, the complete peak assignment of the LC-MS data set is not mandatory and only the differences can be analyzed. All remaining samples can be compared with the sample analyzed as outlined above.

The quantification of a mutant amino acid sequence (variation) is carried out by using one, or some, or all charge states in the mass spectrum relative to each other, whereby for each of the natural amino acid sequence and the mutant amino acid sequence (variation) the same charge state is chosen, the extracted ion chromatograms (EICs) can be generated and the area under the curve of the peaks corresponding to each other in the EICs can be quantified relative to each other according to the following formula:

$$\frac{100}{\text{natural amino acid sequence} + \text{mutant amino acid sequence}} * \text{mutant amino acid sequence}$$

With the method as reported herein a method for identification of amino acid sequence mutations (variants) down to the sub-percentage range is available. The overall sensitivity was determined to be at least about 0.5 % depending on nature of the amino acid sequence fragment. In certain cases the sensitivity could be below 0.5 % (e.g. for amino acid sequence fragments with good chromatography and/or ionization properties). In case studies variations between 0.1 % and 10 % can be detected, in some cases down to 0.02 %. This can be achieved e.g. by a defined combination of software tools needed to achieve significant and reliable results.

In case of detection of amino acid sequence mutations (variations) false positive results can be ruled out by confirming the detection by either:

- isolating the polypeptide containing the amino acid sequence mutation (variation) from the peptide map and performing Edman sequencing,
- synthesizing the peptide with the amino acid mutation (variation) and spiking it into the polypeptide for MS and MS/MS analysis confirming the retention and MS/MS profile,
- confirming the presence by DNA sequencing of the respective sample producing cell clone,
- digestion with a different proteolytic enzyme and analysis of the therewith obtained fragments.

**Example of a spiking experiment:**

The evaluation of the sensitivity for detection of polypeptides with mutations (variations) was performed with a model polypeptide, a monoclonal antibody (mAb), spiked with a second mAb with amino acid sequence mutations (variation) at various ratios, i.e. in various concentrations. In one example, the mAb with amino acid sequence mutation (variation) was spiked at 1 % (w/w), i.e. two different peptides are expected to be identified by the method as reported herein.

| | | |
|---|---|---|
| mAb: | LC peptide | XXI**X**XXX |
| mutant mAb: | LC peptide | XX**V**XXXX |
| mAb: | HC peptide | XXXXXXXXX**T**XXXXX |
| mutant mAb: | HC peptide | XXXXXXXXX**I**XXXXX |

In a second example, the mutant (variant) mAb was spiked at varying ratios (0.5-10 %) to the mAb, i.e. 17 different mutant (variant) peptides were expected.

Thus, herein is reported a method for determining a polypeptide with a mutant amino acid sequence, characterized in comprising the following steps:

a) providing at least two samples of the polypeptide,
b) incubating the samples each with the same protease,
c) analyzing the incubated samples by a two dimensional data analysis using a combination of a reversed phase liquid chromatography separation, and a mass spectrometry analysis and/or MS/MS analysis,
d) defining the data set obtained with one sample in step c) as reference sample and comparing the data sets obtained with the other samples in step c) with the data set of the reference sample, whereby every amino acid sequence difference determined is an amino acid sequence mutation of the polypeptide and thereby determining a polypeptide with a mutant amino acid sequence.

In all embodiments every amino acid sequence difference with a ratio of more than 3 of the intensity of the sample mass spectrum signal to the intensity of the reference mass spectrum signal is an amino acid sequence mutation. In all embodiments the m/z frame width used in the analyzing is 1.6 or more. In a further embodiment the method further comprises a step e) determining the identity and position of the amino acid mutation in the amino acid sequence by MS/MS analysis. In also an embodiment a further sample is provided which comprises the polypeptide spiked with the polypeptide with a known amino acid sequence mutation (variation) and the further sample is incubated and analyzed and compared in addition to the provided samples. In one embodiment the analyzing is performed at a pH value of less than 8.0 and at a temperature of less than 40 °C. In another embodiment the samples are provided in a tris (hydroxymethyl) aminomethane buffer. In also an embodiment the incubating of the samples with a protease is a cleavage of the polypeptide by the protease in amino acid sequence fragments of from 3 to 60 amino acid residues in length. In still an embodiment the comparing in step d) is performed with the data of one or some or all MS charge states. In one embodiment the polypeptide is an immunoglobulin, immunoglobulin fragment or immunoglobulin conjugate. In another embodiment the samples are incubated for 16 hours to 18 hours with the protease and thereafter formic acid or trifluoro acetic acid are added. In a further embodiment the samples are incubated for 4 hours with the protease and thereafter formic acid or trifluoro acetic acid are added. In also an embodiment the comparing comprises overlaying of the mass spectrometric total ion chromatogram (MS-TIC) of the reference sample and each of the other samples to be analyzed, whereby the intensity ratio of all overlapped and aligned masses is calculated, whereby peaks with a ratio of more than 3, especially more than 10, are evaluated for being an amino acid sequence mutation. In still another embodiment the comparing comprises in addition comparing the DNA translated proteolytic fragment peptide pattern of the theoretical amino acid sequence and the total mass spectrometry ion chromatogram (MS-TIC) of the sample to be analyzed, and amino acid sequence mutations are identified by adding to or substracting from, respectively, each calculated theoretical mass of the theoretical amino acid sequence the mass differences resulting from a nucleic acid mutation, deletion, or insertion in a base triplet (codon) with an amino acid change.

A further aspect as reported herein is a method for producing a polypeptide comprising the following step:

- selecting a cell producing a polypeptide, whereby the polypeptide comprises the smallest number or ratio, respectively, of amino acid sequence mutations of all processed samples or with respect to a reference sample or predetermined amino acid sequence determined with a method as reported herein.

Also an aspect as reported herein is a method for producing an immunoglobulin comprising the steps of:

a) providing at least two cells comprising a nucleic acid encoding the immunoglobulin,

b) single depositing and cultivating the cells,

c) performing a method as reported herein,

d) selecting a cell producing an immunoglobulin, whereby the immunoglobulin comprises the smallest number or ratio, respectively, of amino acid sequence mutations with respect to a reference sample,

e) cultivating the cell,

f) producing the polypeptide by recovering the polypeptide from the cell or the cultivation medium.

The following examples, sequence listing and figures are provided to aid the understanding of the present invention, the true scope of which is set forth in the appended claims.

## Description of the Sequence Listing

**SEQ ID NO: 01** Anti-CCR5 antibody heavy chain variable domain 1.
**SEQ ID NO: 02** Anti-CCR5 antibody light chain variable domain 1.
**SEQ ID NO: 03** Anti-CCR5 antibody heavy chain variable domain 2.
**SEQ ID NO: 04** Anti-CCR5 antibody light chain variable domain 2.
**SEQ ID NO: 05** Anti-CCR5 antibody heavy chain variable domain 3.
**SEQ ID NO: 06** Anti-CCR5 antibody light chain variable domain 3.
**SEQ ID NO: 07** Human IgG1 constant region.
**SEQ ID NO: 08** Human IgG4 constant region.
**SEQ ID NO: 09** Human kappa light chain constant domain.
**SEQ ID NO: 10** Human lambda light chain constant domain.

## Description of the Figures

**Figure 1** A representative temporal sequence of the scan event cycle for data dependent acquisition of MS/MS spectra; abbreviations: FT ICR - Fourier transform ion cyclotron resonance; LIT - linear ion trap; CID - collisionally induced dissociation; RP - resolution power; SID - source induced dissociation.

**Figure 2** Total ion chromatogram of tryptic peptide maps from a reference anti-CD 19 antibody (reference) and a sample anti-CD 19 antibody (sample) acquired on an LTQ FT ICR (Thermo Scientific).

**Figure 3** Overlay of LC-MS chromatograms aligned by retention time of prominent peaks. Data were from tryptic peptide maps of two anti-CD 19 antibodies (reference and sample). A and B are mean TIC profiles of two replicates each.

**Figure 4** Scatter Plot showing the differences between sample and reference anti-CD 19 antibody.

**Figure 5** Zoom of Figure 4.

**Figure 6** Assigned MS/MS spectrum of the sample anti-CD 19 antibody HC amino acid sequence fragment (y-ion series and b-ion series of collisionally induced dissociation of the doubly charged parent ion). Characteristic MS/MS fragments for the amino acid sequence variant are shown in **bold.**

**Figure 7** Assigned MS/MS spectrum of the sample anti-CD19 antibody LC amino acid sequence fragment (y-ion series and b-ion series of collisionally induced dissociation of the doubly charged parent ion). Characteristic MS/MS fragments for the amino acid sequence variant are shown in **bold.**

**Figure 8** Extracted ion chromatogram of natural amino acid sequence and the amino acid sequence of the sample anti-CD 19 antibody HC amino acid sequence fragment within the LC-MS sample run. Result of quantitation: 2 wt-% of mutant amino acid sequence (variant) is present in the sample.

**Figure 9** Extracted ion chromatogram of the natural amino acid sequence and the sample anti-CD 19 antibody LC amino acid sequence fragment within the LC-MS sample run. Result of quantitation: 0.5 wt-% of mutant amino acid sequence (variant) is present in the sample.

**Figure 10** Overlay of LC-MS chromatograms aligned by retention time of prominent peaks. Data were from tryptic peptide maps of two anti-CCR5 antibodies (reference and sample).

**Figure 11** Scatter Plot showing the differences between reference and sample anti-CCR5 antibody.

**Figure 12** Extracted ion chromatogram of the natural amino acid sequence and the sample anti-CCR5 antibody LC amino acid fragment within the LC-MS sample run.

**Figure 13** Extracted ion chromatogram of the natural amino acid sequence and the sample anti-CCR5 antibody HC amino acid sequence fragment within the LC-MS sample run.

## Example 1

**Materials and Methods**

**Sample preparation method for the reference and the sample:**

**a) Reduction and alkylation:**

250 μg of immunoglobulin in a volume of maximal 100 μl were diluted **with** denaturation buffer (0.4 M Tris-HCl, 8.0 M guanidinium-hydrochloride, pH 8) to a final volume of 240 μl. 20 μl of dithiothreitol (240 mM in denaturation buffer) were added to the solution and the mixture was incubated at 37 °C ± 2 °C for 60 minutes. Afterwards 20 μl of an iodoacetic acid solution (0.6 M in purified water) were added, vigorously mixed and incubated for 15 min. at room temperature in the dark. The alkylation reaction was stopped by the addition of 30 μl of a dithiothreitol solution (240 mM in denaturation buffer).

**b) Buffer exchange:**

The buffer of 300 μl (approximately 250 μg or 3.2 nmol) of a solution comprising the denatured, reduced, carboxymethylated immunoglobulin was exchanged using a NAP™ 5 Sephadex™ G-25 desalting column. Briefly, the column was equilibrated with 10 ml of a buffer solution comprising 50 mM Tris-HCl, pH 7.5, the sample was applied to the column, the column was washed with 350 μl of the previous buffer solution and the sample was recovered in approximately 480 μl. Between each step (column equilibration, sample application, washing and elution) the solution was allowed to enter the packed column bed completely.

**c) Enzymatic digestion:**

48 μl of a trypsin solution (0.2 g/l in Tris-HCl, pH 7.5) were added to the buffer exchanged immunoglobulin solution and incubated at 37 °C for about 16 hours at room temperature. The digestion was stopped by the addition of 20 μl 10 % (v/v) trifluoro acetic acid (TFA) solution.

**LC-MS/MS data acquisition method:**

The LC-MS/MS analysis was performed by chromatographic separation (LC) of the hydrolytic peptides obtained in the tryptic digestion steps followed by MS and MS/MS detection, respectively, using a nano ESI ion source from Advion BioSciences as an interface between HPLC and mass spectrometer.
The chromatography was carried out with the following parameters:

| | |
|---|---|
| HPLC: | Dionex Ultimate 3000 |
| Flow rate: | 40 μl/min |
| UV detection wavelength: | 220 nm and 280 nm |
| Temperature of the column oven: | 35 °C |
| Sample loop: | 10 μl |
| Column: | Dionex pep Map C18, 3 μm, 100 Å, 1x150 mm |
| Injection volume: | 10 μl |

Eluent A = HPLC gradient grade water containing 0.1 % formic acid
Eluent B = HPLC gradient grade acetonitrile containing 0.1 % formic acid

The applied gradient was from 5 vol% Eluent B to 100 vol% Eluent B in 75 minutes.
The nano ESI MS or MS/MS was carried out with the following parameters:

| | |
|---|---|
| Nano ESI source: | Triversa NanoMate (Advion) |
| Flow into mass spectrometer ion source: | approx. 200 nl/min. managed by a flow splitter |
| Gas pressure: | 0.1 - 0.5 psi |
| Voltage to apply: | 1.1 - 1.7 kV |

(continued)

| Positive ion: | selected |
|---|---|

The mass spectrometric detection was carried out with the following parameters:

| Instrument: | ESI LTQ-FT ICR (Thermo Scientific) |
|---|---|
| Capillary temperature: | 175 °C |
| Ion trap collision energy for MS/MS: | 40 % |
| Tube lens voltage: | 100 V |
| Dynamic exclusion feature: | enabled (Repeat Count: 1, Exclusion Duration: 8 sec, Exclusion mass width: 3 ppm). |

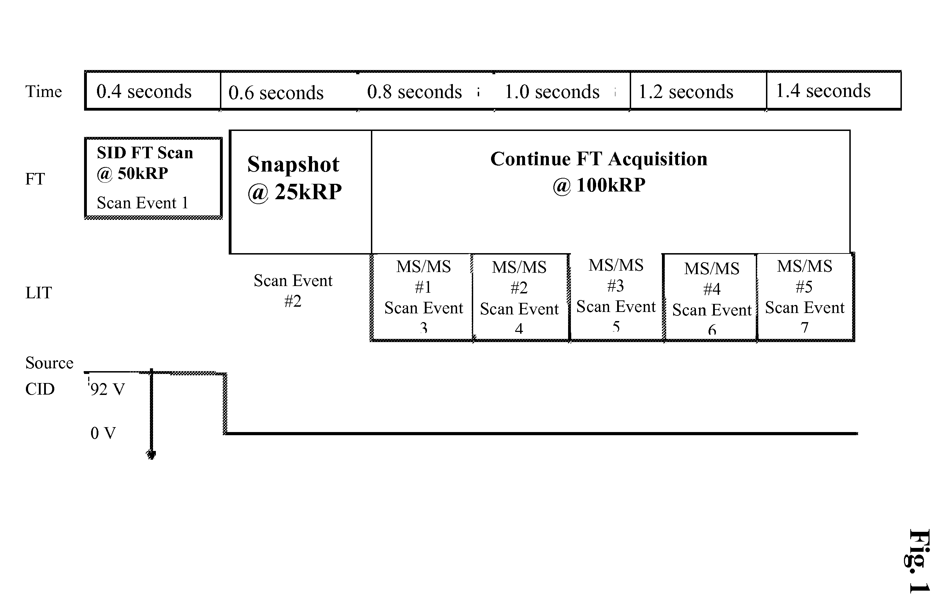

A representative temporal sequence of the scan event cycle for data dependent acquisition of MS/MS spectra is shown in Figure 1.

The data acquisition range was 350 - 2000 m/z for MS spectra. M/Z range for MS/MS spectra was used according to standard instrument settings. The number of MS/MS spectra per high resolution FT scan can vary between 3 to 5. The SID scan is not mandatory for this type of analysis.

## Example 2

### Analysis of an anti-CD19 antibody reference and sample

### Data generation:

The reference and the sample of the anti-CD 19 antibody have been treated according to the Materials and Methods section. MS data has been acquired according to the Materials and Methods section.

### Data analysis:

### a) Detection of differences between reference and sample using LC-MS data sets

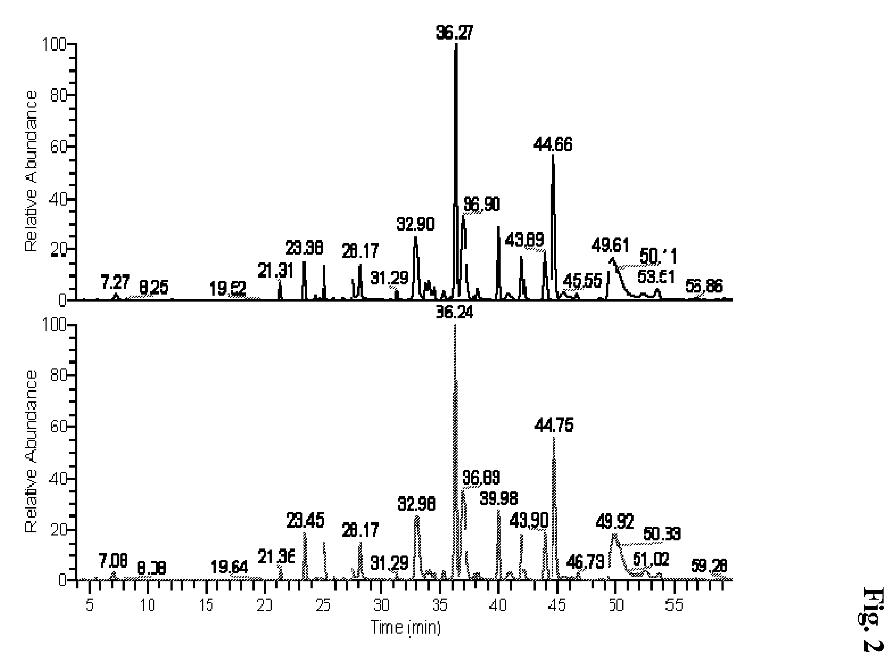

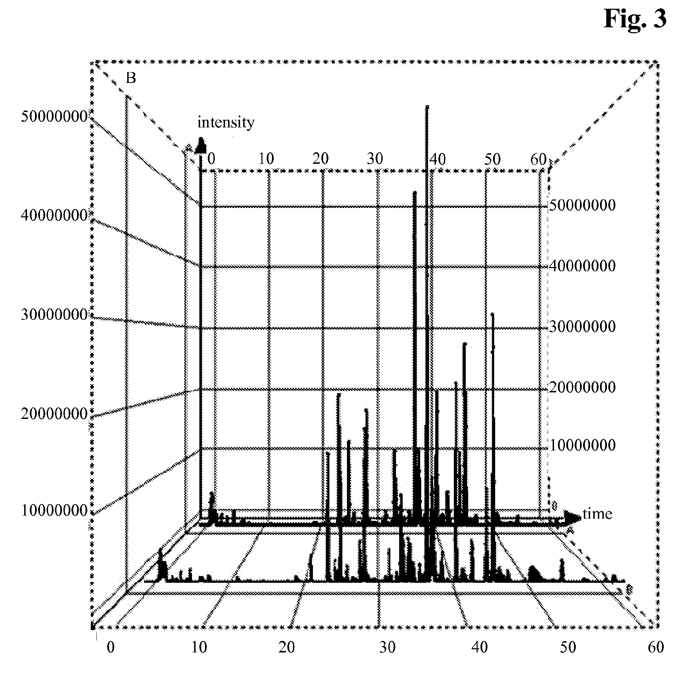

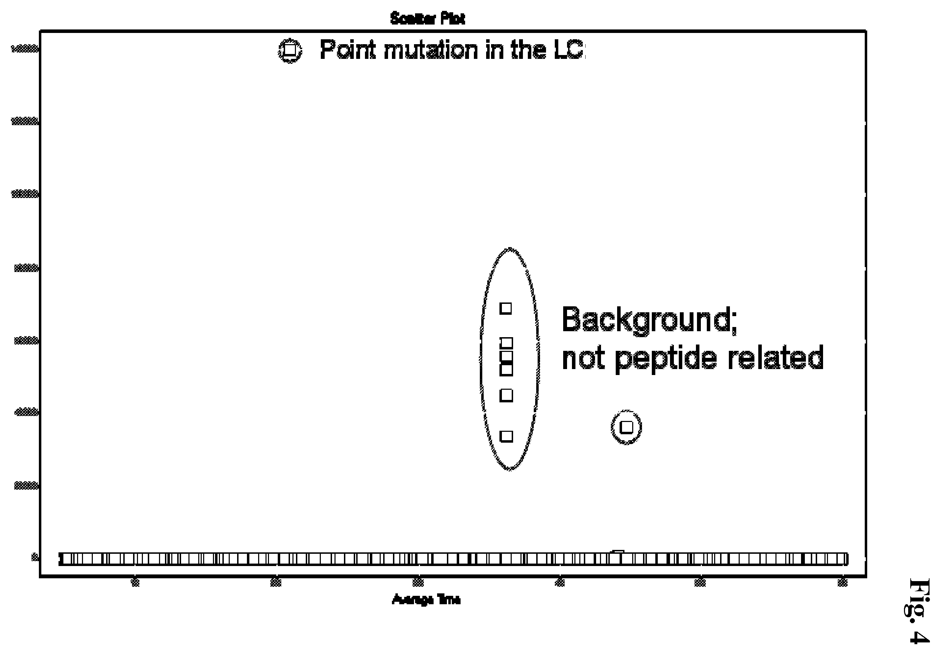

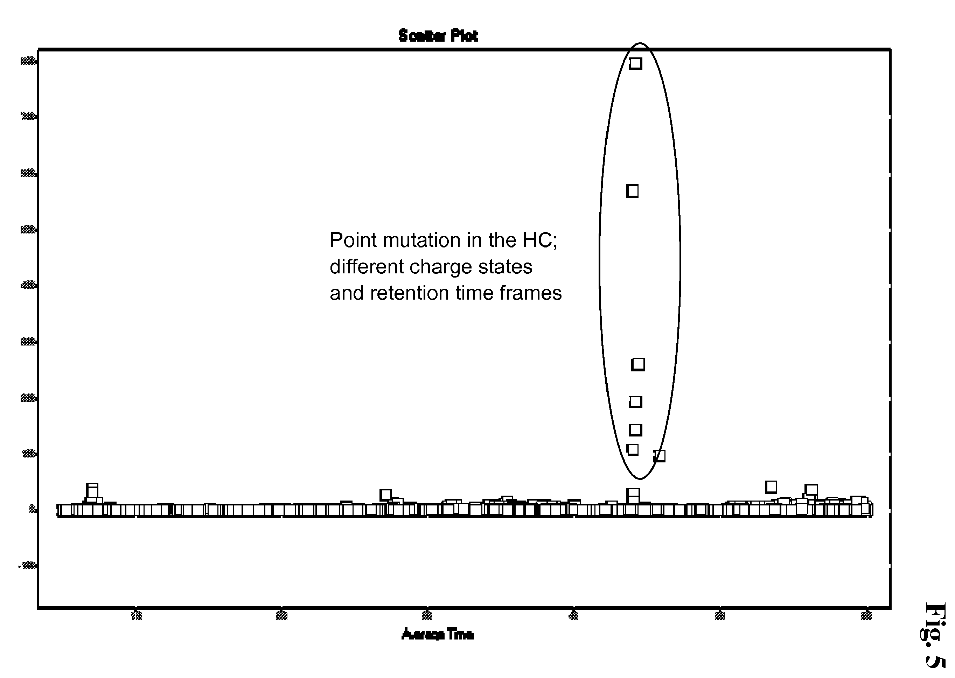

For comparison of mass profiles obtained for the reference and the sample (total ion chromatograms (TICs), see Figure 2) SIEVE™ software package (version 1.1.0 from Thermo Scientific) has been used. Briefly, the TIC data sets of the reference and the sample were aligned by retention time (Figure 3) and compared for mass peak intensities within a preset retention time window down to a preset threshold (Figures 4 and 5).

Mass signals different in reference and sample according to the parameters predefined for evaluation are listed in Table 1. Mass signals present at identical intensities in the reference and the sample appear at a ratio of 1. Mass signals with higher intensity in the sample than in the reference (e.g. amino acid point mutations) appear with ratios larger than 1. The ratios were calculated by dividing the overall signal intensity in the given m/z versus retention time frame of the sample by the corresponding intensity in the reference. If the overall signal intensity in the given m/z versus retention time frame of the reference is zero (e.g. no background signal due to active noise reduction during data acquisition), the ratio is equal to the overall signal intensity of the sample in the corresponding frame (e.g. hits 2-10 in Table 1).

**Table 1:** Data for all differential peaks with a ratio >50.

| # | m/z start | m/z stop | time start | time stop | ratio | manual interpretation |
|---|---|---|---|---|---|---|
| 1 | 1061.19 | 1062.79 | 20.7385 | 21.0385 | 140043 | mutation 1; z = 1 |
| 2 | 1441.94 | 1443.54 | 36.043 | 36.343 | 68943 | background, not peptide related, no MS/MS triggered |
| 3 | 1989.61 | 1991.21 | 36.0909 | 36.3909 | 59552 | background, not peptide related, no MS/MS triggered |
| 4 | 1864.12 | 1865.72 | 36.0909 | 36.3909 | 55943 | background, not peptide related, no MS/MS triggered |

(continued)

| # | m/z start | m/z stop | time start | time stop | ratio | manual interpretation |
|---|---|---|---|---|---|---|
| 5 | 890.645 | 892.245 | 36.0909 | 36.3909 | 52340 | background, not peptide related, no MS/MS triggered |
| 6 | 1718.96 | 1720.56 | 36.0909 | 36.3909 | 52235 | background, not peptide related, no MS/MS triggered |
| 7 | 1347.59 | 1349.19 | 36.0909 | 36.3909 | 45291 | background, not peptide related, no MS/MS triggered |
| 8 | 1110.76 | 1112.36 | 36.0909 | 36.3909 | 45107 | background, not peptide related, no MS/MS triggered |
| 9 | 1235.23 | 1236.83 | 44.5509 | 44.8509 | 36205 | background, not peptide related, no MS/MS triggered |
| 10 | 965.611 | 967.211 | 36.0909 | 36.3909 | 33871 | background, not peptide related, no MS/MS triggered |
| 11 | 606.874 | 608.474 | 44.0258 | 44.3258 | 798 | mutation 2; z = 3 |
| 12 | 607.21 | 608.81 | 43.8741 | 44.1741 | 571 | mutation 2; z = 3 |
| 13 | 606.875 | 608.475 | 44.2013 | 44.5013 | 261 | mutation 2; z = 3 |
| 14 | 910.713 | 912.313 | 44.0258 | 44.3258 | 194 | mutation 2; z = 2 |
| 15 | 607.879 | 609.479 | 44.0258 | 44.3258 | 143 | mutation 2; z = 3 |
| 16 | 910.218 | 911.818 | 43.8741 | 44.1741 | 108 | mutation 2;, z = 2 |
| 17 | 601.538 | 603.138 | 45.6276 | 45.9276 | 96 | not identified |
| 18 | 735.059 | 736.659 | 53.367 | 53.667 | 42 | present in both LC-MS runs but with only slightly different intensity |
| 19 | 1022.27 | 1023.87 | 6.84557 | 7.14557 | 40 | present in both LC-MS runs but with only slightly different intensity |

Parameters used for comparison of reference and sample LC-MS data with SIEVE were as follows:

Frame m/z width:      1.6
Frame time width:     0.3 min
Intensity threshold:  10000
M/z start:            350
M/z end:              2000
Search peak width:    30 %
Retention time start: 5 min.
Retention time stop:  60 min.

These parameters have been optimized for distinguishing amino acid sequence difference related hits from false positive, i.e. non-amino acid sequence difference related, hits. They only need to be adjusted according to the actual parameters of the LC-MS data as they are:

i) chromatographic resolution (frame time width), and
ii) the sensitivity of the instrument used and the background noise (intensity threshold).

Further the required sensitivity of the method, e.g. for the detection of very low abundant mutant amino acid sequence (variants), determines the intensity threshold to be set. Using e.g. a LTQ FT ICR instrument mutant sequences (variants) down to 0.2 % absolute frequency have been identified.

**b) Identification of differences between reference and sample using MS/MS data**

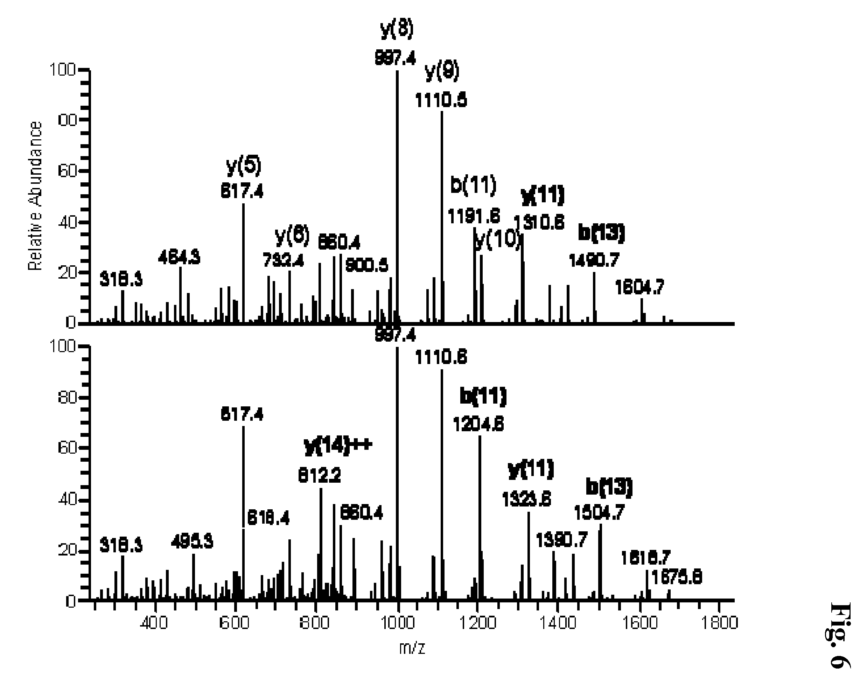

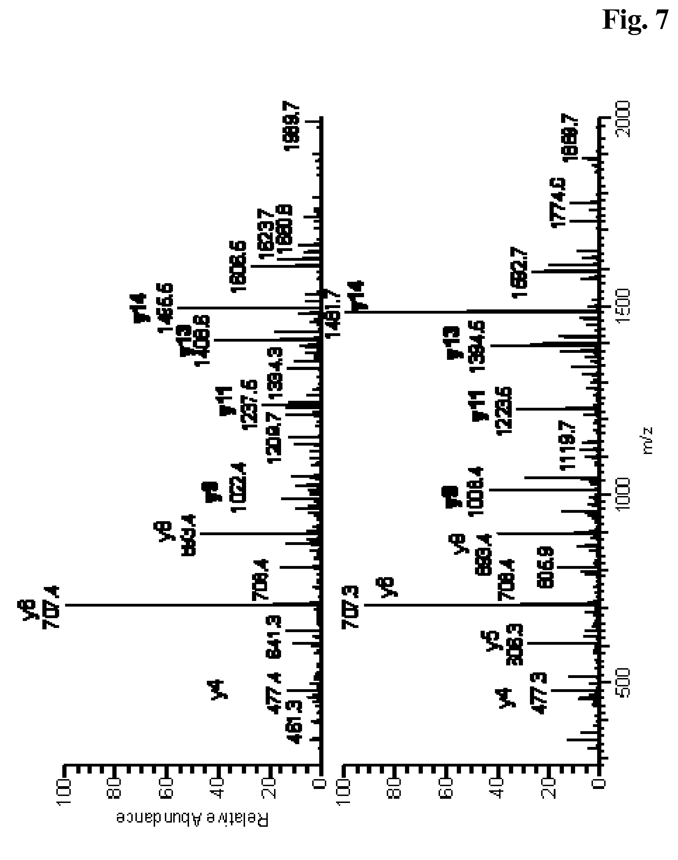

For identification of the differences found using the procedure as described in the previous paragraph first of all the isotope peak cluster was checked for being typical for peptides. Then, it was checked whether the respective m/z signal was selected for generation of MS/MS fragmentation and whether it was identified by the Mascot error tolerant search (Mascot ETS). Each tentative sequence variant identified by Mascot ETS was checked and confirmed manually using the obtained MS/MS fragment ion spectra (see Figure 6 and Figure 7).

Alternatively, if MS/MS data have been recorded and Mascot ETS did not propose a sequence de novo sequencing was applied either manually or by using software.

**c) Quantitation of identified mutant amino acid sequences (variants)**

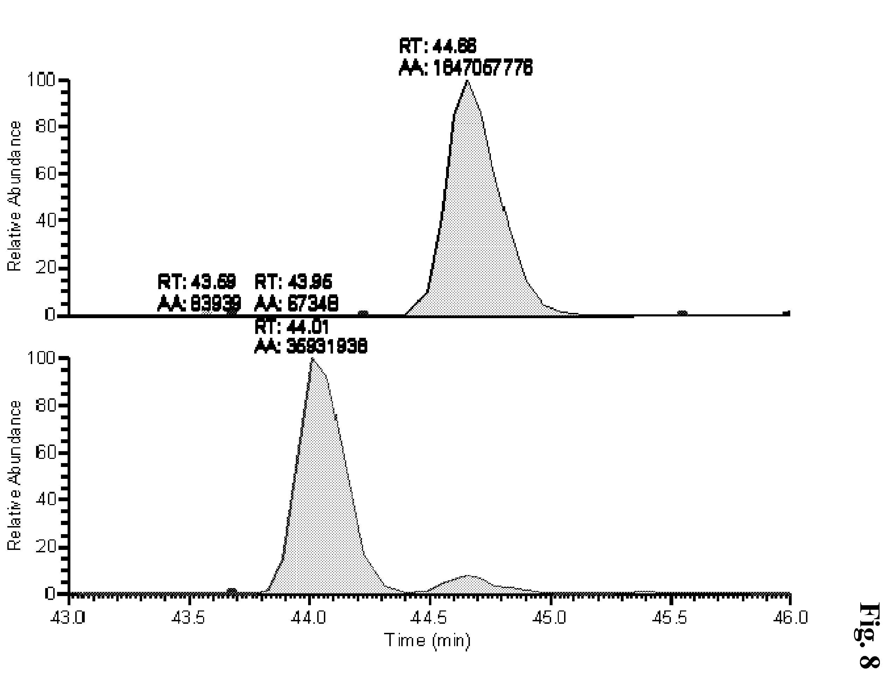

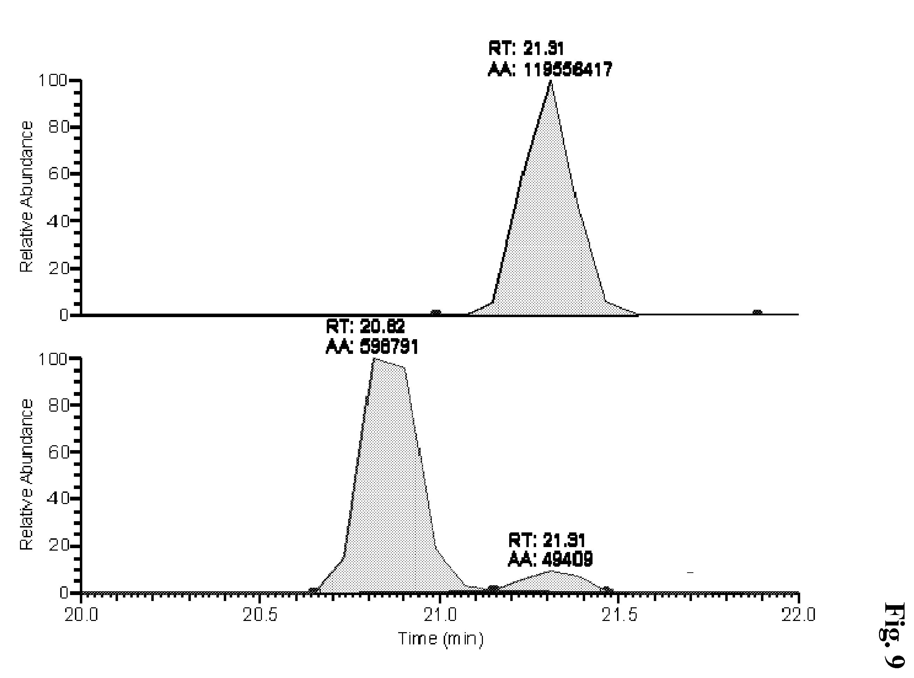

The identified mutant amino acid sequence (variant) was quantified at the level of the (tryptic) amino acid sequence fragment containing the mutation (variation) and in relation to the original, non-mutated amino acid sequence fragment within the same sample (see Figure 8 and Figure 9). Two extracted ion chromatograms (EIC) were generated from the LC-MS data of the sample, one for the sample and one for the reference sample. The extracted ion chromatograms (EICs) include all charge states and all isotopic peaks of the respective peptide. The peaks generated by the respective EICs were integrated using the instrument software (XCalibur) and the areas calculated hereby are used in the following formula:

$$\text{Percentage (sequence variation)} = \frac{100}{\text{natural amino acid sequence} + \text{mutant amino acid sequence}} * \text{mutant amino acid sequence}$$

Wherein the peak area of the extracted ion chromatogram taking into account all charge states and all isotopic peaks is used.

**Example 3**

**Analysis of an anti-CCR5 antibody reference and sample**

For the determination of a single amino acid change (mutation) in the variable domains, i.e. light chain variable domain and heavy chain variable domain, an anti-CCR5 antibody has been employed. The first or reference anti-CCR5 antibody has a variable heavy chain and variable light chain domain amino acid sequence selected from the pairs of SEQ ID NO: 01 and 02, SEQ ID NO: 03 and 04, and SEQ ID NO: 05 and 06. The second or sample anti-CCR5 antibody has the following amino acid mutations (changes): in the heavy chain variable domain (VH) the isoleucine residue at amino acid position 109 is mutated (changed) to threonine (VH-I109T), in the light chain variable domain (VL) the valine residue at amino acid position 52 is mutated (changed) to isoleucine (VL-V52I).

The sample anti-CCR5 antibody has been spiked at 1 wt-% to the reference anti-CCR5 antibody.

**a) Detection of differences between reference and sample amino acid sequences using LC-MS data sets**

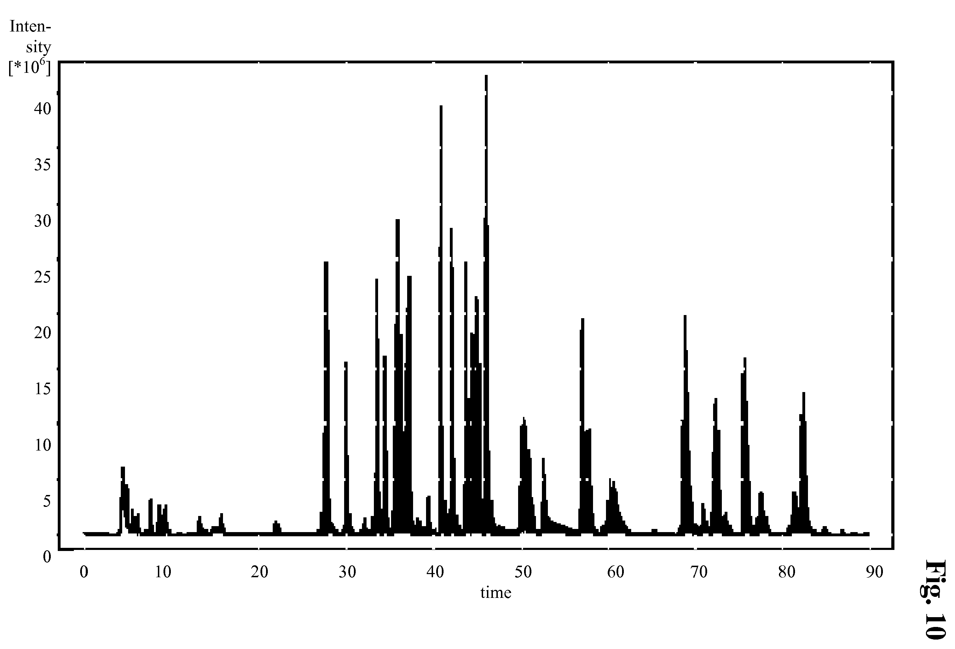

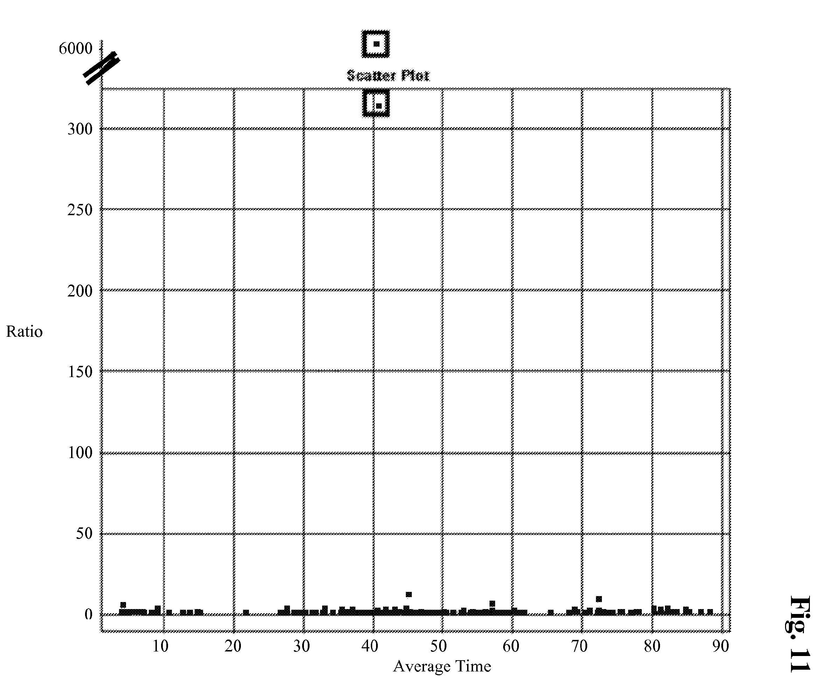

For comparison of mass profiles obtained for the reference and the sample (total ion chromatograms (TICs)) SIEVE™ software package (version 1.1.0 from Thermo Scientific) has been used. Briefly, the data sets of the reference and the sample were aligned chromatographically by retention time (Figure 10) and compared for mass peak intensities within a preset retention time window down to a preset threshold (Figure 11).

Mass signals differences in reference and sample according to the parameters predefined for evaluation are listed in Table 2. Mass signals present at identical intensities in the reference and the sample appear at a ratio of 1. Mass signals with higher intensity in the sample than in the reference (e.g. amino acid point mutations) appear with ratios larger than 1. The ratios were calculated by dividing the overall signal intensity in the given m/z versus retention time frame of the sample by the corresponding intensity in the reference. If the overall signal intensity in the given m/z versus retention time frame of the reference is zero (e.g. no background signal due to active noise reduction during data acquisition), the ratio is equal to the overall signal intensity of the sample in the corresponding frame.

**Table 2:** Data for differential peaks.

| # | m/z start | m/z stop | time start | time stop | ratio | manual interpretation |
|---|-----------|----------|------------|-----------|-------|-----------------------|
| 1 | 947.913 | 947.933 | 39.6552 | 42.1552 | 429464 | mutation |
| 2 | 948.414 | 948.434 | 39.6873 | 42.1873 | 312.837 | mutation |

**b) Identification of differences between reference and sample using MS/MS data**

For identification of the differences found using the procedure as described in the previous paragraph first of all the isotope peak cluster was checked for being typical for peptides. Then, it was checked whether the respective m/z signal was selected for generation of MS/MS fragmentation and whether it was identified by the Mascot error tolerant search (Mascot ETS). Each tentative amino acid sequence mutation (variant) identified by Mascot ETS was checked and confirmed manually using the obtained MS/MS fragment ion spectra.

Alternatively, if MS/MS data have been recorded and Mascot ETS did not propose a sequence de novo sequencing was applied either manually or by using software.

**c) Quantitation of identified sequence variants**

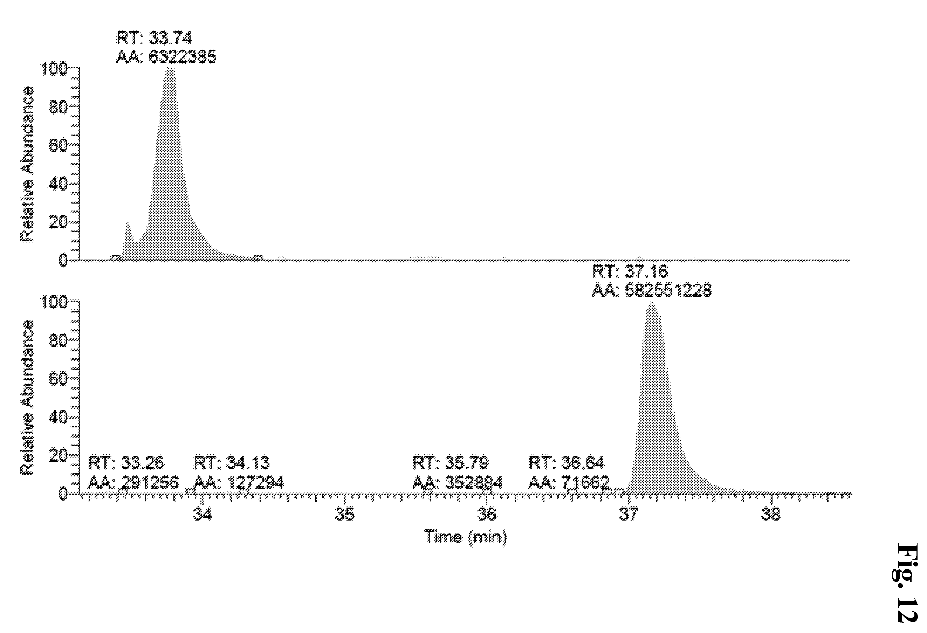

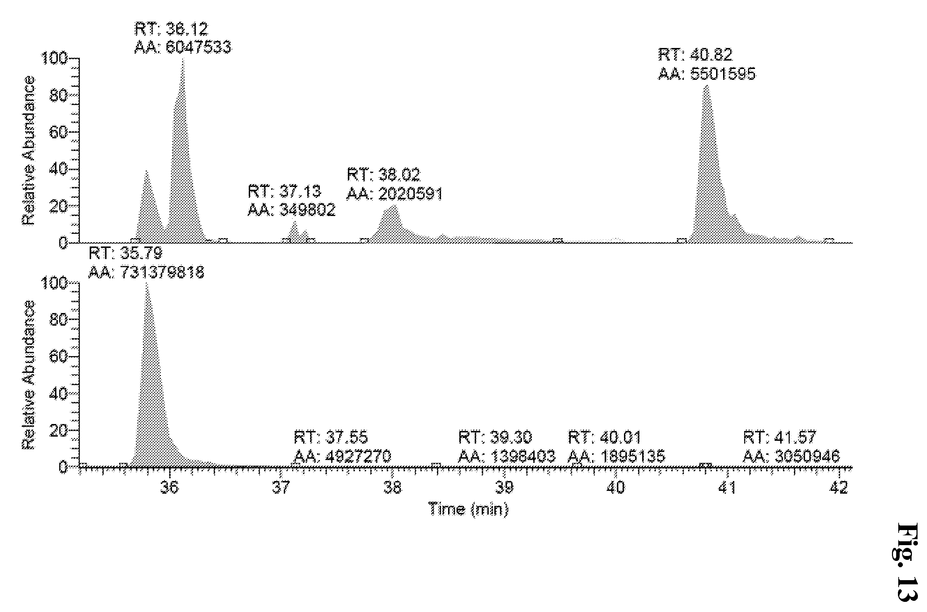

The identified mutant amino acid sequence (variant) was quantified at the level of the (tryptic) amino acid sequence fragment containing the mutation (variation) and in relation to the original, non-mutated peptide within the same sample (see Figure 12 and Figure 13). Two extracted ion chromatograms (EIC) were generated from the LC-MS data of the sample, one for the mutant peptide (variant) and one for the native peptide. The extracted ion chromatograms (EICs) include all charge states and all isotopic peaks of the respective peptide. The peaks generated by the respective EICs were integrated using the instrument software (XCalibur) and the areas calculated hereby according to the formula as shown in Example 1.

## Claims

1. A method for determining a polypeptide with a mutant amino acid sequence, **characterized in** comprising the following steps:

   a) providing at least two samples of the polypeptide,
   b) incubating the samples each with the same protease,
   c) analyzing the incubated samples by a two dimensional data analysis using a combination of a reversed phase liquid chromatography separation and a mass spectrometry analysis and/or MS/MS analysis,
   d) defining the data set obtained with one sample in step c) as reference sample and comparing the data sets obtained with the other samples in step c) with the data set of the reference sample, whereby every amino acid sequence difference with a ratio of more than 3 of the intensity of the sample mass spectrum signal to the intensity of the reference mass spectrum signal is an amino acid sequence mutation of the polypeptide and thereby determining a polypeptide with a mutant amino acid sequence,

   wherein the m/z frame width is 1.6 or more for pattern comparison.

2. The method according to any one of the preceding claims further comprising a step:

   e) determining the identity and position of the amino acid mutations in the amino acid sequence by MS/MS analysis.

3. The method according to any one of the preceding claims, **characterized in that** a further sample is provided which comprises the polypeptide spiked with the polypeptide with a known amino acid sequence mutation and the further sample is incubated and analyzed and compared in addition to the provided samples.

4. The method according to any one of the preceding claims, **characterized in that** the sample preparation is performed at a pH value of less than pH 8.0 and at a temperature of less than 40 °C.

5. The method according to any one of the preceding claims, **characterized in that** the samples are provided in a tris (hydroxymethyl) aminomethane buffer.

6. The method according to any one of the preceding claims, **characterized in that** the incubating of the samples with a protease is a cleavage of the polypeptide by the protease in amino acid sequence fragments of from 3 to 60 amino acid residues in length.

7. The method according to any one of the preceding claims, **characterized in that** the comparing in step d) is performed with the data of one or some or all MS charge states.

8. The method according to any one of the preceding claims, **characterized in that** the polypeptide is an immunoglobulin, immunoglobulin fragment or immunoglobulin conjugate.

9. The method according to any one of the preceding claims, **characterized in that** the samples are incubated for 16 hours to 18 hours with the protease and thereafter formic acid or trifluoro acetic acid are added.

10. The method according to any one of claims 1 to 8, **characterized in that** the samples are incubated for 4 hours with the protease and thereafter formic acid or trifluoro acetic acid are added.

11. The method according to any one of the preceding claims, **characterized in that** the comparing comprises overlaying of the mass spectrometric total ion chromatogram (MS-TIC) of the reference sample and each of the other samples to be analyzed,
whereby the intensity ratio of all overlapped and aligned masses is calculated, whereby peaks with a ratio of more than 10 are evaluated for being an amino acid sequence mutation.

12. The method according to any one of the preceding claims, **characterized in that** the comparing comprises in addition comparing the DNA translated proteolytic fragment peptide pattern of the theoretical amino acid sequence and the total mass spectrometry ion chromatogram (MS-TIC) of the sample to be analyzed, and amino acid sequence mutations are identified by adding to or substracting from, respectively, each calculated theoretical mass of the theoretical amino acid sequence the mass differences resulting from a nucleic acid mutation, deletion, or insertion in a base triplet (codon) with an amino acid change.

**Patentansprüche**

1. Verfahren zum Bestimmen eines Polypeptids mit einer mutierten Aminosäuresequenz, **dadurch gekennzeichnet, dass** es die folgenden Schritte umfasst:

    a) Bereitstellen mindestens zweier Proben des Polypeptids,
    b) Inkubieren jeder der Proben mit der gleichen Protease,
    c) Analysieren der inkubierten Proben mittels einer zweidimensionalen Datenanalyse unter Verwendung einer Kombination von einer Auftrennung durch Umkehrphasen-Flüssigchromatographie und einer Massenspektrometrieanalyse und/oder MS/MS-Analyse,
    d) Definieren des mit einer Probe in Schritt c) erhaltenen Datensatzes als Referenzprobe und Vergleichen der mit den übrigen Proben in Schritt c) erhaltenen Datensätze mit dem Datensatz der Referenzprobe, wobei jeder Aminosäuresequenzunterschied mit einem Verhältnis von mehr als 3 der Intensität des Massenspektrumsignals der Probe gegenüber der Intensität des Massenspektrumsignals der Referenz eine Aminosäuresequenzmutation des Polypeptids ist und wodurch ein Polypeptid mit einer mutierten Aminosäuresequenz bestimmt wird, wobei die m/z-Rahmenbreite (m/z frame width) für den Vergleich der Muster 1,6 oder mehr ist.

2. Verfahren nach einem der vorangehenden Ansprüche, umfassend des Weiteren einen Schritt:

    e) Bestimmen der Identität und Position der Aminosäuremutationen in der Aminosäuresequenz durch MS/MS-Analyse.

3. Verfahren nach einem der vorangehenden Ansprüche, **dadurch gekennzeichnet, dass** eine weitere Probe bereitgestellt wird, die das Polypeptid umfasst, das mit dem Polypeptid mit einer bekannten Aminosäuresequenzmutation versetzt ist, und dass die weitere Probe inkubiert und analysiert wird und zusätzlich mit den bereitgestellten Proben verglichen wird.

4. Verfahren nach einem der vorangehenden Ansprüche, **dadurch gekennzeichnet, dass** die Herstellung der Probe bei einem pH-Wert von weniger als pH 8,0 und einer Temperatur von weniger als 40°C durchgeführt wird.

5. Verfahren nach einem der vorangehenden Ansprüche, **dadurch gekennzeichnet, dass** die Proben in einem Tris-(hydroxymethyl)-aminomethan-Puffer bereitgestellt werden.

6. Verfahren nach einem der vorangehenden Ansprüche, **dadurch gekennzeichnet, dass** das Inkubieren der Proben mit einer Protease ein Spalten des Polypeptids durch die Protease in Aminosäuresequenzfragmente mit einer Länge von 3 bis 60 Aminosäureresten ist.

7. Verfahren nach einem der vorangehenden Ansprüche, **dadurch gekennzeichnet, dass** der Vergleich in Schritt d) mit den Daten eines oder einiger oder aller MS-Ladungszustände durchgeführt wird.

8. Verfahren nach einem der vorangehenden Ansprüche, **dadurch gekennzeichnet, dass** das Polypeptid ein Immunglobulin, Immunglobulinfragment oder Immunglobulinkonjugat ist.

9. Verfahren nach einem der vorangehenden Ansprüche, **dadurch gekennzeichnet, dass** die Proben 16 Stunden bis 18 Stunden mit der Protease inkubiert werden und anschließend Ameisensäure oder Trifluoressigsäure hinzugefügt wird.

10. Verfahren nach einem der Ansprüche 1 bis 8, **dadurch gekennzeichnet, dass** die Proben 4 Stunden mit der Protease inkubiert werden und anschließend Ameisensäure oder Trifluoressigsäure hinzugefügt wird.

11. Verfahren nach einem der vorangehenden Ansprüche, **dadurch gekennzeichnet, dass** der Vergleich das Überlagern des massenspektorgraphischen Gesamtionenchromatogramms (MS-TIC) der Referenzprobe und jeder der übrigen zu analysierenden Proben umfasst, wodurch das Intensitätsverhältnis aller sich überlagernden und zur Deckung gebrachten Massen (overlapped and aligned masses) berechnet wird, wodurch Peaks mit einem Verhältnis von mehr als 10 als Aminosäuresequenzmutation bewertet werden.

12. Verfahren nach einem der vorangegangenen Ansprüche, **dadurch gekennzeichnet, dass** der Vergleich zusätzlich das Vergleichen des DNAtranslatierten proteolytischen Peptidfragment-Musters der theoretischen Aminosäuresequenz und des massenspektrographischen Gesamtionenchromatogramms (MS-TIC) der zu analysierenden Probe umfasst, und dass Aminosäuresequenzmutationen identifiziert werden durch Addieren bzw. Subtrahieren der Massenunterschiede, die sich aus Nucleinsäuremutation, -deletion oder -insertion in einem Basentriplet (Codon) mit einem Aminosäurewechsel ergeben, von jeder berechneten theoretischen Masse der theoretischen Aminosäuresequenz.

**Revendications**

1. Procédé pour déterminer un polypeptide avec une séquence d'aminoacides mutante, **caractérisé en ce qu'**il comprend les étapes suivantes :

   a) la fourniture d'au moins deux échantillons du polypeptide,
   b) la mise en incubation des échantillons chacun avec la même protéase,
   c) l'analyse des échantillons mis en incubation par une analyse de données bidimensionnelle en utilisant une combinaison de séparation par chromatographie en milieu liquide à phase inversée et une analyse par spectrométrie de masse et/ou une analyse MS/MS,
   d) la définition de la série de résultats obtenue avec un échantillon de l'étape c) comme échantillon de référence et la comparaison des séries de résultats obtenues avec les autres échantillons dans l'étape c) avec la série de résultats de l'échantillon de référence, chaque différence de séquence d'aminoacides avec un rapport de plus de 3 de l'intensité du signal de spectre de masse de l'échantillon à l'intensité du signal de spectre de masse de référence étant ainsi une mutation de séquence d'aminoacides du polypeptide et déterminant de ce fait un polypeptide avec une séquence d'aminoacides mutante,

dans lequel la largeur de cadre m/z est égale ou supérieure à 1,6 pour la comparaison de motifs.

2. Procédé suivant l'une quelconque des revendications précédentes, comprenant en outre une étape :

e) de détermination de l'identité et de la position des mutations d'aminoacides dans la séquence d'aminoacides par analyse MS/MS.

3. Procédé suivant l'une quelconque des revendications précédentes, **caractérisé en ce qu'**il est fourni un échantillon supplémentaire qui comprend le polypeptide additionné d'une petite quantité du polypeptide avec une mutation de séquence d'aminoacides connue et **en ce que** l'échantillon supplémentaire est mis en incubation, analysé et comparé en plus des échantillons fournis.

4. Procédé suivant l'une quelconque des revendications précédentes, **caractérisé en ce que** la préparation d'échantillon est effectuée à une valeur de pH inférieure à pH 8,0 et à une température inférieure à 40°C.

5. Procédé suivant l'une quelconque des revendications précédentes, **caractérisé en ce que** les échantillons sont fournis dans un tampon au tris(hydroxyméthyl)aminométhane.

6. Procédé suivant l'une quelconque des revendications précédentes, **caractérisé en ce que** la mise en incubation des échantillons avec une protéase est un clivage du polypeptide par la protéase en fragments de séquence d'aminoacides de 3 à 60 résidus d'aminoacides de longueur.

7. Procédé suivant l'une quelconque des revendications précédentes, **caractérisé en ce que** la comparaison dans l'étape d) est effectuée avec les résultats d'un, d'une partie ou de la totalité des états de charge de MS.

8. Procédé suivant l'une quelconque des revendications précédentes, **caractérisé en ce que** le polypeptide est une immunoglobuline, un fragment d'immunoglobuline ou un conjugué d'immunoglobuline.

9. Procédé suivant l'une quelconque des revendications précédentes, **caractérisé en ce que** les échantillons sont mis en incubation pendant 16 heures à 18 heures avec la protéase, puis de l'acide formique ou l'acide trifluoro-acétique est ajouté.

10. Procédé suivant l'une quelconque des revendications 1 à 8, **caractérisé en ce que** les échantillons sont mis en incubation pendant 4 heures avec la protéase, puis de l'acide formique ou l'acide trifluoroacétique est ajouté.

11. Procédé suivant l'une quelconque des revendications précédentes, **caractérisé en ce que** la comparaison comprend la superposition du chromatogramme ionique total en spectrométrie de masse (MS-TIC) de l'échantillon de référence et de chacun des autres échantillons à analyser,
le rapport des intensités de toutes les masses superposées et alignées étant ainsi calculé, ce qui fait que les pics avec un rapport supérieur à 10 sont évalués en tant que mutation de séquence d'aminoacides.

12. Procédé suivant l'une quelconque des revendications précédentes, **caractérisé en ce que** la comparaison comprend en outre la comparaison du motif peptidique de fragments protéolytiques de traduction d'ADN de la séquence d'aminoacides théorique et du chromatogramme ionique en spectrométrie de masse (MS-TIC) total de l'échantillon à analyser, et des mutations de séquence d'aminoacides sont identifiées en ajoutant à, ou en soustrayant de, respectivement, chaque masse théorique calculée de la séquence d'aminoacides théorique les différences de masses résultant d'une mutation, délétion ou insertion d'acide nucléique dans un triplet de bases (codon) avec un changement d'aminoacides.

| Time | 0.4 seconds | 0.6 seconds | 0.8 seconds | 1.0 seconds | 1.2 seconds | 1.4 seconds |
|---|---|---|---|---|---|---|

**FT**

| **SID FT Scan @ 50kRP** Scan Event 1 | **Snapshot @ 25kRP** | **Continue FT Acquisition @ 100kRP** |
|---|---|---|

**LIT**

| Scan Event #2 | MS/MS #1 Scan Event 3 | MS/MS #2 Scan Event 4 | MS/MS #3 Scan Event 5 | MS/MS #4 Scan Event 6 | MS/MS #5 Scan Event 7 |
|---|---|---|---|---|---|

Source CID

92 V

0 V

**Fig. 1**

**Fig. 2**

Fig. 3

EP 2 537 035 B1

Scatter Plot

○ Point mutation in the LC

Background;
not peptide related

Average Time

Fig. 4

**Fig. 5**

Scatter Plot

Point mutation in the HC;
different charge states
and retention time frames

Access Time

Fig. 6

Fig. 7

Fig. 8

**Fig. 9**

**Fig. 10**

Fig. 11

**Fig. 12**

**Fig. 13**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 10001645 A

### Non-patent literature cited in the description

- **BARNES, C.A.S. et al.** *Mass. Spectrom. Rev.,* 2007, vol. 26, 370-388
- **ZHANG, Z. et al.** *Mass. Spectrom. Rev.,* 2009, vol. 28, 147-176
- **YU, X.C. et al.** *Anal. Chem.,* 2009, vol. 81, 9282-9290
- **HOUDE, D. et al.** *J. Chromatogr.,* 2006, vol. 1123, 189-198
- **LEBKOWSKI, J. S. et al.** *Mol. Cell Biol.,* 1984, vol. 4, 1951-1960

SEQUENCE LISTING

<110> F. Hoffmann-La Roche AG

<120> Method for the determination of sequence variants of polypeptides

<130> 26601 WO

<150> EP10001645.0
<151> 2010-02-18

<160> 10

<170> PatentIn version 3.5

<210> 1
<211> 117
<212> PRT
<213> Mus musculus

<400> 1

```
Gln Val Gln Leu Lys Gln Ser Gly Pro Gly Leu Val Arg Pro Ser Gln
1               5               10                  15

Ser Leu Ser Ile Thr Cys Thr Val Ser Gly Phe Pro Leu Gly Val Phe
            20                  25                  30

Gly Val His Trp Val Arg Gln Ser Pro Gly Lys Gly Leu Glu Trp Leu
            35                  40                  45

Gly Val Ile Trp Lys Gly Gly Asn Thr Asp Tyr Asn Ala Ala Phe Met
        50                  55                  60

Ser Arg Leu Arg Ile Ser Lys Asp Asn Ser Lys Ser Gln Val Phe Phe
65                  70                  75                  80

Arg Met Asn Ser Leu Gln Thr Asp Asp Thr Ala Ile Tyr Tyr Cys Ala
                85                  90                  95

Lys Val Asn Leu Ala Asp Ala Met Asp Tyr Trp Gly Gln Gly Thr Ser
            100                 105                 110

Val Ile Val Ser Ser
            115
```

<210> 2
<211> 108
<212> PRT
<213> Mus musculus

<400> 2

```
Asp Ile Gln Met Thr Gln Ser Pro Ala Ser Leu Ser Ala Ser Val Gly
1               5               10                  15
```

```
Glu Thr Val Thr Ile Thr Cys Arg Ser Ser Gly Asn Ile His Gly Tyr
        20              25              30

Leu Ala Trp Phe Gln Gln Lys Gln Gly Lys Ser Pro Gln Leu Leu Val
        35              40              45

Tyr Asn Thr Lys Ala Leu Ala Glu Gly Val Pro Ser Arg Phe Ser Gly
        50              55              60

Ser Gly Ser Gly Thr Gln Phe Ser Leu Lys Ile Asn Asn Leu Gln Pro
65              70              75              80

Glu Asp Phe Gly Ile Tyr Tyr Cys Gln His His Tyr Asp Leu Pro Arg
                85              90              95

Thr Phe Gly Gly Gly Thr Lys Leu Glu Ile Lys Arg
        100             105
```

<210> 3
<211> 117
<212> PRT
<213> Mus musculus

<400> 3

```
Gln Val Gln Leu Lys Gln Ser Gly Pro Gly Leu Val Arg Pro Ser Gln
1               5               10              15

Ser Leu Ser Ile Thr Cys Thr Val Ser Gly Phe Pro Leu Gly Ile Phe
        20              25              30

Gly Val His Trp Val Arg Gln Ser Pro Gly Lys Gly Leu Glu Trp Leu
        35              40              45

Gly Val Ile Trp Lys Gly Gly Asn Thr Asp Tyr Asn Ala Ala Phe Met
        50              55              60

Ser Arg Leu Arg Ile Thr Lys Asp Asn Ser Lys Ser Gln Val Phe Phe
65              70              75              80

Arg Met Asn Ser Leu Gln Thr Asp Asp Thr Ala Ile Tyr Tyr Cys Ala
                85              90              95

Lys Val Asn Leu Ala Asp Ala Met Asp Tyr Trp Gly Gln Gly Thr Ser
        100             105             110

Val Ile Val Ser Ser
        115
```

<210> 4
<211> 108
<212> PRT
<213> Mus musculus

<400> 4

```
Asp Ile Gln Met Thr Gln Ser Pro Ala Ser Leu Ser Ala Ser Val Gly
1               5                   10                  15

Glu Thr Val Thr Ile Thr Cys Arg Ala Ser Gly Asn Ile His Gly Tyr
            20                  25                  30

Leu Ala Trp Phe Gln Gln Lys Gln Gly Lys Ser Pro Gln Leu Leu Val
            35                  40                  45

Tyr Asn Thr Lys Thr Leu Ala Glu Gly Val Pro Ser Arg Phe Ser Gly
        50                  55                  60

Ser Gly Ser Gly Thr Gln Phe Ser Leu Lys Ile Asn Ser Leu Gln Pro
65                  70                  75                  80

Glu Asp Phe Gly Asn Tyr Tyr Cys Gln His His Tyr Asp Leu Pro Arg
                85                  90                  95

Thr Phe Gly Gly Gly Thr Lys Leu Glu Ile Lys Arg
            100                 105
```

<210> 5
<211> 117
<212> PRT
<213> Mus musculus

<400> 5

```
Gln Val Gln Leu Lys Gln Ser Gly Pro Gly Leu Val Arg Pro Ser Gln
1               5               10                  15

Ser Leu Ser Ile Thr Cys Thr Val Ser Gly Phe Pro Leu Gly Thr Phe
            20                  25                  30

Gly Val His Trp Val Arg Gln Ser Pro Gly Lys Gly Leu Glu Trp Leu
            35                  40                  45

Gly Val Ile Trp Arg Gly Gly Asn Thr Asp Tyr Asn Ala Ala Phe Met
        50                  55                  60

Ser Arg Leu Arg Ile Thr Lys Asp Asn Ser Lys Ser Gln Val Phe Phe
65                  70                  75                  80

Arg Met Asn Ser Leu Gln Thr Asp Asp Thr Ala Ile Tyr Tyr Cys Ala
                85                  90                  95

Lys Val Asn Leu Ala Asp Ala Met Asp Tyr Trp Gly Gln Gly Thr Ser
                100                 105                 110

Val Ile Val Ser Ser
            115
```

<210> 6
<211> 108
<212> PRT
<213> Mus musculus

<400> 6

```
Asp Ile Gln Met Thr Gln Ser Pro Ala Ser Leu Ser Ala Ser Val Gly
1               5               10              15

Glu Thr Val Thr Ile Thr Cys Arg Ala Ser Gly Asn Ile His Gly Tyr
            20              25              30

Leu Ala Trp Phe Gln Gln Lys Gln Gly Lys Ser Pro Gln Leu Leu Val
            35              40              45

Tyr Asn Thr Lys Thr Leu Ala Glu Gly Val Pro Ser Arg Phe Ser Gly
    50              55              60

Ser Gly Ser Gly Thr Gln Phe Ser Leu Lys Ile Asn Ser Leu Gln Pro
65              70              75              80

Glu Asp Phe Gly Asn Tyr Tyr Cys Gln His His Tyr Asp Leu Pro Arg
                85              90              95

Thr Phe Gly Gly Gly Thr Lys Leu Glu Ile Lys Arg
            100             105
```

<210> 7
<211> 330
<212> PRT
<213> Homo sapiens

<400> 7

```
Ala Ser Thr Lys Gly Pro Ser Val Phe Pro Leu Ala Pro Ser Ser Lys
1               5               10              15

Ser Thr Ser Gly Gly Thr Ala Ala Leu Gly Cys Leu Val Lys Asp Tyr
            20              25              30

Phe Pro Glu Pro Val Thr Val Ser Trp Asn Ser Gly Ala Leu Thr Ser
            35              40              45

Gly Val His Thr Phe Pro Ala Val Leu Gln Ser Ser Gly Leu Tyr Ser
    50              55              60
```

```
Leu Ser Ser Val Val Thr Val Pro Ser Ser Ser Leu Gly Thr Gln Thr
65              70              75              80

Tyr Ile Cys Asn Val Asn His Lys Pro Ser Asn Thr Lys Val Asp Lys
                85              90              95

Lys Val Glu Pro Lys Ser Cys Asp Lys Thr His Thr Cys Pro Pro Cys
            100             105             110

Pro Ala Pro Glu Leu Leu Gly Gly Pro Ser Val Phe Leu Phe Pro Pro
            115             120             125

Lys Pro Lys Asp Thr Leu Met Ile Ser Arg Thr Pro Glu Val Thr Cys
            130             135             140

Val Val Val Asp Val Ser His Glu Asp Pro Glu Val Lys Phe Asn Trp
145             150             155             160

Tyr Val Asp Gly Val Glu Val His Asn Ala Lys Thr Lys Pro Arg Glu
                165             170             175

Glu Gln Tyr Asn Ser Thr Tyr Arg Val Val Ser Val Leu Thr Val Leu
            180             185             190

His Gln Asp Trp Leu Asn Gly Lys Glu Tyr Lys Cys Lys Val Ser Asn
            195             200             205

Lys Ala Leu Pro Ala Pro Ile Glu Lys Thr Ile Ser Lys Ala Lys Gly
    210             215             220

Gln Pro Arg Glu Pro Gln Val Tyr Thr Leu Pro Pro Ser Arg Asp Glu
225             230             235             240

Leu Thr Lys Asn Gln Val Ser Leu Thr Cys Leu Val Lys Gly Phe Tyr
                245             250             255

Pro Ser Asp Ile Ala Val Glu Trp Glu Ser Asn Gly Gln Pro Glu Asn
                260             265             270

Asn Tyr Lys Thr Thr Pro Pro Val Leu Asp Ser Asp Gly Ser Phe Phe
                275             280             285

Leu Tyr Ser Lys Leu Thr Val Asp Lys Ser Arg Trp Gln Gln Gly Asn
    290             295             300

Val Phe Ser Cys Ser Val Met His Glu Ala Leu His Asn His Tyr Thr
305             310             315             320

Gln Lys Ser Leu Ser Leu Ser Pro Gly Lys
```

325                    330

<210> 8
<211> 327
<212> PRT
<213> Homo sapiens

<400> 8

```
Ala Ser Thr Lys Gly Pro Ser Val Phe Pro Leu Ala Pro Cys Ser Arg
1               5                   10                  15


Ser Thr Ser Glu Ser Thr Ala Ala Leu Gly Cys Leu Val Lys Asp Tyr
            20                  25                  30


Phe Pro Glu Pro Val Thr Val Ser Trp Asn Ser Gly Ala Leu Thr Ser
        35                  40                  45


Gly Val His Thr Phe Pro Ala Val Leu Gln Ser Ser Gly Leu Tyr Ser
    50                  55                  60


Leu Ser Ser Val Val Thr Val Pro Ser Ser Ser Leu Gly Thr Lys Thr
65                  70                  75                  80


Tyr Thr Cys Asn Val Asp His Lys Pro Ser Asn Thr Lys Val Asp Lys
                85                  90                  95


Arg Val Glu Ser Lys Tyr Gly Pro Pro Cys Pro Ser Cys Pro Ala Pro
            100                 105                 110


Glu Phe Leu Gly Gly Pro Ser Val Phe Leu Phe Pro Pro Lys Pro Lys
        115                 120                 125


Asp Thr Leu Met Ile Ser Arg Thr Pro Glu Val Thr Cys Val Val Val
    130                 135                 140


Asp Val Ser Gln Glu Asp Pro Glu Val Gln Phe Asn Trp Tyr Val Asp
145                 150                 155                 160


Gly Val Glu Val His Asn Ala Lys Thr Lys Pro Arg Glu Glu Gln Phe
                165                 170                 175


Asn Ser Thr Tyr Arg Val Val Ser Val Leu Thr Val Leu His Gln Asp
            180                 185                 190


Trp Leu Asn Gly Lys Glu Tyr Lys Cys Lys Val Ser Asn Lys Gly Leu
        195                 200                 205


Pro Ser Ser Ile Glu Lys Thr Ile Ser Lys Ala Lys Gly Gln Pro Arg
    210                 215                 220
```

22

```
Glu Pro Gln Val Tyr Thr Leu Pro Pro Ser Gln Glu Glu Met Thr Lys
225             230             235             240

Asn Gln Val Ser Leu Thr Cys Leu Val Lys Gly Phe Tyr Pro Ser Asp
                245             250             255

Ile Ala Val Glu Trp Glu Ser Asn Gly Gln Pro Glu Asn Asn Tyr Lys
            260             265             270

Thr Thr Pro Pro Val Leu Asp Ser Asp Gly Ser Phe Phe Leu Tyr Ser
            275             280             285

Arg Leu Thr Val Asp Lys Ser Arg Trp Gln Glu Gly Asn Val Phe Ser
    290             295             300

Cys Ser Val Met His Glu Ala Leu His Asn His Tyr Thr Gln Lys Ser
305             310             315             320

Leu Ser Leu Ser Leu Gly Lys
                325
```

<210> 9
<211> 107
<212> PRT
<213> Homo sapiens

<400> 9

```
Arg Thr Val Ala Ala Pro Ser Val Phe Ile Phe Pro Pro Ser Asp Glu
1               5               10              15

Gln Leu Lys Ser Gly Thr Ala Ser Val Val Cys Leu Leu Asn Asn Phe
            20              25              30

Tyr Pro Arg Glu Ala Lys Val Gln Trp Lys Val Asp Asn Ala Leu Gln
            35              40              45

Ser Gly Asn Ser Gln Glu Ser Val Thr Glu Gln Asp Ser Lys Asp Ser
        50              55              60

Thr Tyr Ser Leu Ser Ser Thr Leu Thr Leu Ser Lys Ala Asp Tyr Glu
65              70              75              80

Lys His Lys Val Tyr Ala Cys Glu Val Thr His Gln Gly Leu Ser Ser
            85              90              95

Pro Val Thr Lys Ser Phe Asn Arg Gly Glu Cys
            100             105
```

<210> 10
<211> 105
<212> PRT
<213> Human

<400> 10

```
Gln Pro Lys Ala Ala Pro Ser Val Thr Leu Phe Pro Pro Ser Ser Glu
1               5                   10                  15

Glu Leu Gln Ala Asn Lys Ala Thr Leu Val Cys Leu Ile Ser Asp Phe
        20                  25                  30

Tyr Pro Gly Ala Val Thr Val Ala Trp Lys Ala Asp Ser Ser Pro Val
        35                  40                  45

Lys Ala Gly Val Glu Thr Thr Thr Pro Ser Lys Gln Ser Asn Asn Lys
        50                  55                  60

Tyr Ala Ala Ser Ser Tyr Leu Ser Leu Thr Pro Glu Gln Trp Lys Ser
65                  70                  75                  80

His Arg Ser Tyr Ser Cys Gln Val Thr His Glu Gly Ser Thr Val Glu
                85                  90                  95

Lys Thr Val Ala Pro Thr Glu Cys Ser
        100                 105
```